# EUROPEAN PATENT APPLICATION

(11) **EP 4 434 435 A1**
(43) Date of publication of application: **25.09.2024**
(21) Application number: 21964733.6
(22) Date of filing: 18.11.2021
(51) Int. Cl.: A61B 1/045

(54) **INFORMATION PROCESSING DEVICE, INFORMATION PROCESSING METHOD, AND RECORDING MEDIUM**

(71) Applicant: NEC Corporation, 108-8001 Tokyo (JP)
(72) Inventor: KAMIJO, Kenichi, Tokyo 108-8001 (JP); TAKAHASHI, Ikuma, Tokyo 108-8001 (JP); OKUTSU, Motoyasu, Tokyo 108-8001 (JP); OHTSUKA, Shota, Tokyo 108-8001 (JP); KIMURA, Tatsu, Tokyo 108-8001 (JP)
(74) Representative: Betten & Resch
(86) International application number: PCT/JP2021/042367
(87) International publication number: WO 2023/089718

(57) **Abstract**

The image acquisition means acquires a captured image by an endoscope. The position acquisition means acquires positional information of the endoscope. The lesion detection means detects a lesion from the captured image. The washing degree determination means determines a degree of intestinal tract washing from the captured image. The reaching determination means determines that the endoscope reaches a cecum from the captured image. The end determination means determines an end timing of an examination by the endoscope. The calculation means calculates an observation time based on a timing that the endoscope reaches the cecum and the end timing. The report generation means generates an examination report including the observation time, the degree of intestinal tract washing, a presence or absence of reaching the cecum of the endoscope, and a detection result of the lesion including the positional information of the lesion. The generated examination report is displayed on the display device.

## Description

### TECHNICAL FIELD

The present disclosure relates to processing of information relating to an endoscopic examination.

### BACKGROUND ART

The popularization of AI (Artificial Intelligence) for detecting the lesion from images and moving images captured at the time of the endoscopic examination advances, and the popularization of Al for analyzing the examination qualities and the grade of malignancy of the lesion is also expected in future. For example, Patent Document 1 proposes a method of measuring the time spent by the observation in the endoscopic examination, in order to prove the quality of the examination. Further, Patent Document 2 proposes a method for automatically diagnosing positive or negative or the severity level of the lesion from the endoscopic image of the large intestine.

### PRECEDING TECHNICAL REFERENCES

### PATENT DOCUMENT

Patent Document 1: International Publication WO2020/070818
Patent Document 2: Japanese Patent Application Laid-Open under No. JP 2021-112593

### SUMMARY

### PROBLEM TO BE SOLVED

In the future, it is desired to reflect the examination result and the analysis result of the lesion using AI in the report efficiently.

It is an object of the present disclosure to provide an information processing device capable of automatically reflecting various types of information related to examinations analyzed by AI in the report.

### MEANS FOR SOLVING THE PROBLEM

According to an example aspect of the present disclosure, there is provided an information processing device comprising:
an image acquisition means configured to acquire a captured image by an endoscope;
a position acquisition means configured to acquire positional information of the endoscope;
a lesion detection means configured to detect a lesion from the captured image;
a washing degree determination means configured to determine a degree of intestinal tract washing from the captured image;
a reaching determination means configured to determine that the endoscope reaches a cecum from the captured image;
an end determination means configured to determine an end timing of an examination by the endoscope;
a calculation means configured to calculate an observation time based on a timing that the endoscope reaches the cecum and the end timing; and
a report generation means configured to generate an examination report including the observation time, the degree of intestinal tract washing, a presence or absence of reaching the cecum of the endoscope, and a detection result of the lesion including the positional information of the lesion.

According to another example aspect of the present disclosure, there is provided an information processing method comprising:
acquiring a captured image by an endoscope;
acquiring positional information of the endoscope;
detecting a lesion from the captured image;
determining a degree of intestinal tract washing from the captured image;
determining that the endoscope reaches a cecum from the captured image;
determining an end timing of an examination by the endoscope;
calculating an observation time based on a timing that the endoscope reaches the cecum and the end timing; and
generating an examination report including the observation time, the degree of intestinal tract washing, a presence or absence of reaching the cecum of the endoscope, and a detection result of the lesion including the positional information of the lesion.

According to still another example aspect of the present disclosure, there is provided a recording medium storing a program, the program causing a computer to execute processing of:
acquiring a captured image by an endoscope;
acquiring positional information of the endoscope;
detecting a lesion from the captured image;
determining a degree of intestinal tract washing from the captured image;
determining that the endoscope reaches a cecum from the captured image;
determining an end timing of an examination by the endoscope;
calculating an observation time based on a timing that the endoscope reaches the cecum and the end timing; and
generating an examination report including the observation time, the degree of intestinal tract washing, a presence or absence of reaching the cecum of the endoscope, and a detection result of the lesion including the positional information of the lesion.

### EFFECT

According to the present disclosure, it is possible to efficiently reflect the analysis result of AI in endoscopic examination in the report.

### BRIEF DESCRIPTION OF THE DRAWINGS

[FIG. 1] FIG. 1 is a block diagram showing a schematic configuration of an endoscopic examination system of the first example embodiment.
[FIG. 2] FIG. 2 is a block diagram showing a hardware configuration of an image processing device.
[FIG. 3] FIG. 3 is a block diagram showing a functional configuration of the image processing device.
[FIG. 4] FIG. 4 schematically shows a structure of a large intestine.
[FIG. 5] FIG. 5 shows a display example of an examination report.
[FIG. 6] FIG. 6 is a flowchart of display processing by an image processing device.
[FIG. 7] FIG. 7 is a block diagram showing a functional configuration of an information processing device of a second example embodiment.
[FIG. 8] FIG. 8 is a flowchart of processing by the information processing device of the second example embodiment.

### EXAMPLE EMBODIMENTS

Preferred example embodiments of the present disclosure will be described with reference to the accompanying drawings.

### <First example embodiment>

### [System configuration]

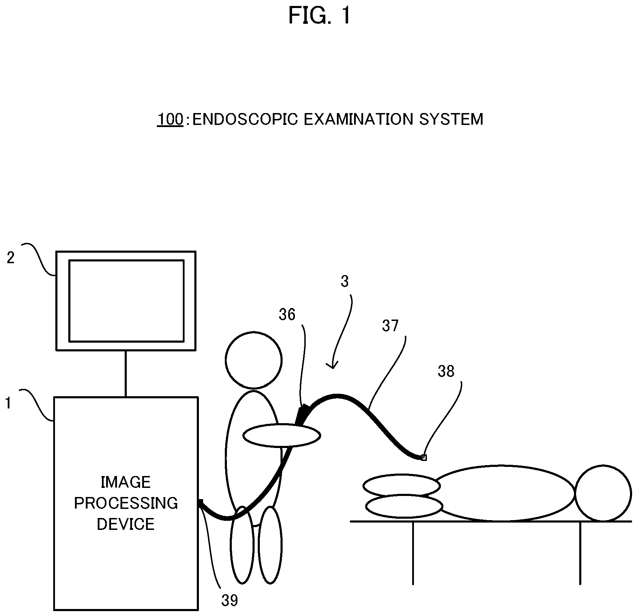

FIG. 1 shows a schematic configuration of an endoscopic examination system 100. At the time of examination (including treatment) using the endoscope, the endoscopic examination system 100 collects information of examination quality analyzed by AI and detects and analyzes lesions using AI. Then, the endoscopic examination system 100 displays the examination quality and the analysis result of the lesions in the report after the end of the examination using the endoscope. Thus, the subjects for endoscopy and examiners can easily grasp information for the examination quality and the lesions analyzed by AI.

As shown in FIG. 1, the endoscopic examination system 100 mainly includes an image processing device 1, a display device 2, and an endoscope 3 connected to the image processing device 1.

The image processing device 1 acquires a moving image (hereinafter also referred to as an "endoscopic video Ic") captured by the endoscope 3 during the endoscopic examination from the endoscope 3 and displays display data for the check by the examiner of the endoscopic examination on the display device 2. Specifically, the image processing device 1 acquires a moving image of organs captured by the endoscope 3 as an endoscopic video Ic during the endoscopic examination. In addition, when the examiner finds a lesion during the endoscopic examination, he or she operates the endoscope 3 to input a photographing instruction of the lesion position. Based on the photographing instruction by the examiner, the image processing device 1 generates a lesion image capturing the lesion position. Specifically, the image processing device 1 generates the lesion image which is a still image, from the endoscopic image Ic which is a moving image, on the basis of the photographing instruction of the examiner.

The display device 2 is a display or the like for displaying images on the basis of the display signal supplied from the image processing device 1.

The endoscope 3 mainly includes an operation unit 36 used by the examiner to input instructions such as air supply, water supply, angle adjustment, and the photographing instruction, a shaft 37 having flexibility and inserted into an organ of a subject to be examined, a tip portion 38 with a built-in image-taking unit such as an ultra-compact imaging element, and a connection unit 39 for connection with the image processing device 1.

While the following explanation is mainly given on the processing of endoscopic examination for a large intestine, the subjects of examination may be gastrointestinal (digestive organs) such as the stomach, esophagus, small intestine, and duodenum, as well as the large intestine.

In addition, the portion to be detected in the endoscopic examination is not limited to the lesion portion, but may be any portion in which the examiner needs attention (also referred to as a "target portion"). Such a target portion may be, for example, the lesion portion, a portion where irritation has occurred, a portion where a surgical wound or other cut has occurred, a portion where folds or protrusions has occurred, or a portion where the tip portion 38 of the endoscope 3 is likely to contact (easily stuck) at the wall surface of the intraluminal region.

### [Hardware configuration]

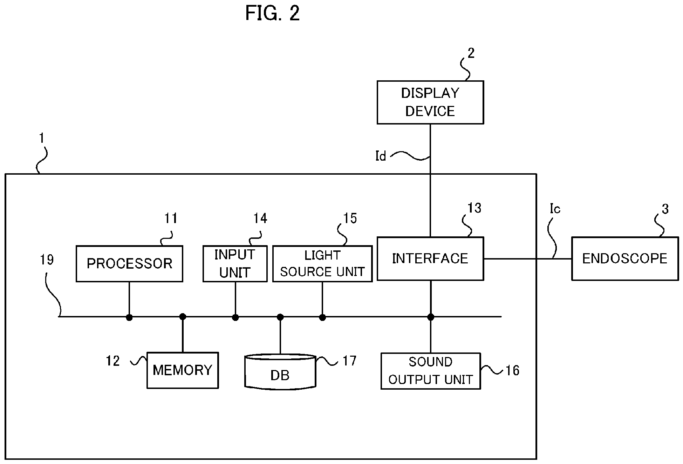

FIG. 2 shows a hardware configuration of the image processing device 1. The image processing device 1 mainly includes a processor 11, a memory 12, an interface 13, an input unit 14, a light source unit 15, a sound output unit 16, and a data base (hereinafter referred to as "DB") 17. These elements are connected with each other via a data bus 19.

The processor 11 executes a predetermined processing by executing a program stored in the memory 12. The processor 11 is a processor such as a CPU (Central Processing Unit), a GPU (Graphics Processing Unit), and a TPU (Tensor Processing Unit). The processor 11 may be configured by multiple processors. The processor 11 is an example of a computer.

The memory 12 is configured by various volatile memories used as a working memory and non-volatile memories for storing information needed for the processing of the image processing device 1, such as a RAM (Random Access Memory) and a ROM (Read Only Memory). Incidentally, the memory 12 may include an external storage device such as a hard disk connected to or incorporated in the image processing device 1, and may include a storage medium such as a removable flash memory or a disk medium. The memory 12 stores a program for the image processing device 1 to execute processing in the present example embodiment.

Also, the memory 12 temporarily stores a series of endoscopic videos Ic taken by the endoscope 3 in the endoscopic examination, based on the control of the processor 11. Further, the memory 12 temporarily stores the lesion images photographed in response to the photographing instructions by the examiner during the endoscopic examination. These images are stored in the memory 12 in association with, for example, subject identification information (e.g., the patient ID) and time stamp information, etc.

The interface 13 performs an interface operation between the image processing device 1 and the external devices. For example, the interface 13 supplies the display data Id generated by the processor 11 to the display device 2. Also, the interface 13 supplies the illumination light generated by the light source unit 15 to the endoscope 3. Also, the interface 13 supplies an electrical signal indicating the endoscopic video Ic supplied from the endoscope 3 to the processor 11. The interface 13 may be a communication interface such as a network adapter for wired or wireless communication with an external device, or may be a hardware interface compliant with a USB (Universal Serial Bus), SATA (Serial Advanced Technology Attachment), etc.

The input unit 14 generates an input signal based on the operation of the examiner. The input unit 14 is, for example, a button, a touch panel, a remote controller, a voice input device, or the like. The light source unit 15 generates the light to be delivered to the tip portion 38 of the endoscope 3. The light source unit 15 may also incorporate a pump or the like for delivering water or air to be supplied to the endoscope 3. The sound output unit 16 outputs the sound based on the control of the processor 11.

The DB 17 stores the endoscopic images acquired by past endoscopic examinations of the subject, and the lesion information. The lesion information includes lesion image and information related to the lesion (hereinafter referred to as "related information"). The DB 17 may include an external storage device, such as a hard disk connected to or incorporated in the image processing device 1, and may include a storage medium, such as a removable flash memory. Instead of providing the DB 17 in the endoscopic examination system 100, the DB 17 may be provided in an external server or the like to acquire associated information from the server through communication.

### [Functional configuration]

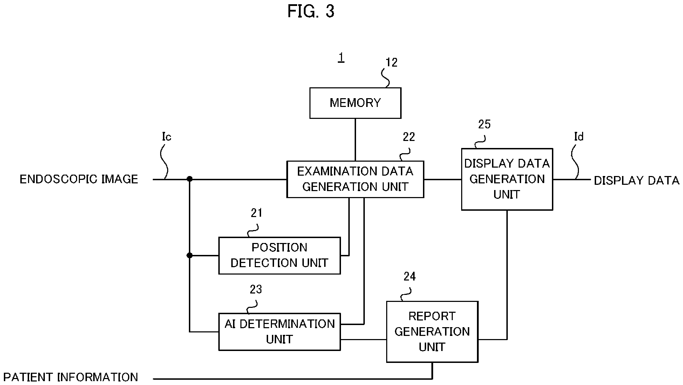

FIG. 3 is a block diagram showing a functional configuration of the image processing device 1. The image processing device 1 functionally includes a position detection unit 21, an examination data generation unit 22, an AI determination unit 23, a report generation unit 24, and a display data generation unit 25.

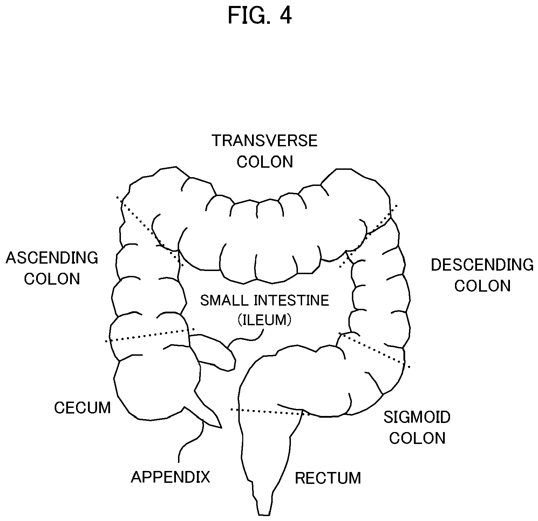

The image processing device 1 receives the endoscopic image Ic from the endoscope 3. The endoscopic image Ic is inputted into the position detection unit 21, the examination data generation unit 22, and the AI determination unit 23. The position detection unit 21 detects the position of the endoscope 3, i.e., the imaging position of the endoscopic image, based on the endoscopic image Ic. Specifically, the position detection unit 21 detects the imaging position by image analysis of the inputted endoscopic image Ic. Here, the imaging position may be three-dimensional coordinates in the organ of the examination target, but may be at least an information indicating one of a plurality of regions in the organ of the examination target. For example, as shown in FIG. 4, the large intestine includes multiple sites such as the cecum, the ascending colon, the transverse colon, the descending colon, the sigmoid colon, and the rectum. Therefore, when the examination target is the large intestine, the position detection unit 21 may detect whether at least the imaging position belongs to any of the above-described regions.

Specifically, the position detection unit 21 can estimate which region of the large intestine the imaging position at that time belongs to, based on the pattern of the mucous membrane, the presence or absence of the folds, the shape of the folds in the endoscopic image, the number of the folds passed by the movement of the endoscope 3, and the like. The position detection unit 21 may estimate the imaging position by estimating the movement speed of the endoscope 3 based on the endoscopic image and calculating the movement distance in the large intestine based on the movement speed and the time. In addition to the image analysis of the endoscopic image, the position detection unit 21 may detect the imaging position using the insertion length of the endoscope 3 inserted into the organ. In the present example embodiment, the detection method of the imaging position in the organ of the examination target is not limited to a specific method. The position detection unit 21 outputs the detected imaging position to the examination data generation unit 22.

The examination data generation unit 22 generates the examination data of the imaging position based on the endoscopic image Ic and the imaging position detected by the position detection unit 21, and temporarily stores the generated examination data in the memory 12. Specifically, when the examiner operates the endoscope 3, the examination data generation unit 22 generates the examination data including the endoscopic image Ic captured by the endoscope 3 and imaging information including the examination date and time, the imaging position, the lesion image, and the like, and stores the examination data in the memory 12. The examination data generation unit 22 outputs the generated examination data to the AI determination unit 23, and the display data generation unit 25.

The AI determination unit 23 performs image analysis on the endoscopic image Ic to determine whether or not lesions exist. The AI determination unit 23 detects lesion-like portions included in the endoscopic image using an image recognition model prepared in advance. When detecting the lesion-like portion, the AI determination unit 23 generates the lesion image that is the still image. Then, the AI determination unit 23 determines the lesion size, the grade of malignancy of the lesion, and so on from the lesion image. The AI determination unit 23 acquires the lesion image generated on the basis of the photographing instruction of the examiner from the examination data generated by the examination data generation unit 22, and may determine the lesion size, the grade of malignancy of the lesion, and so on from the lesion image. The AI determination unit 23 outputs the lesion image and the lesion determination result to the examination data generation unit 22, and stores them in the memory 12. Furthermore, the AI determination unit 23 outputs the lesion image and the lesion determination result to the report generation unit 24.

In addition, the AI determination unit 23 performs image analysis on the endoscopic image Ic to detect the ileocecal valve or the appendiceal orifice. The AI determination unit 23 determines that the endoscope has reached the cecum when detecting the ileocecal valve or the appendiceal orifice. Then, the AI determination unit 23 measures the elapsed time from the start of the examination to the end of the examination. Here, the timing of the start of the examination is, for example, the timing at which the AI determination unit 23 determines that the endoscope has reached the cecum. The timing of the end of the examination may be the timing when the AI determination unit 23 detects that the endoscope has moved out of the body based on the endoscopic image Ic, or the timing when the examiner operates the examination ending button.

Furthermore, during the endoscopic examination, the AI determination unit 23 measures the time of treatment (hereinafter, referred to as "treatment time") such as the excision of the polyp, the hemostasis, and the biopsy. The AI determination unit 23 calculates the time (hereinafter, referred to as "observation time") by subtracting the treatment time from the elapsed time from the start of the examination to the end of the examination. The AI determination unit 23 also performs image analysis on the endoscopic image Ic to detect the residue, and determines the degree of intestinal tract washing.

The AI determination unit 23 outputs the determination result of reaching the cecum, the observation time, and the determination result of the degree of intestinal tract washing to the examination data generation unit 22 to store it in the memory 12. Furthermore, the AI determination unit 23 outputs the determination result of reaching the cecum, the observation time, and the determination result of the degree of intestinal tract washing to the report generation unit 24.

In addition, the image processing device 1 receives patient information of the patient who is the subject, through the input unit 14. The patient information is information that uniquely identifies the patient, and may be a patient name, an ID uniquely assigned to each patient or a personal identification number such as a My Number. The patient information is inputted to the report generation unit 24.

The report generation unit 24 determines the examination reliability based on the determination result of reaching the cecum, the observation time, and the determination result of the degree of intestinal tract washing, which are inputted from the AI determination unit 23. The report generation unit 24 generates the report data on the basis of the data inputted from the AI determination unit 23, the patient information, and the examination reliability. The report generation unit 24 outputs the generated report data to the display data generation unit 25.

The display data generation unit 25 generates the display data Id using the examination data inputted from the examination data generation unit 22 and the report data inputted from the report generation unit 24, and outputs the display data to the display device 2.

### [Display example]

Next, a display example by the display device 2 will be described.

### (Examination report)

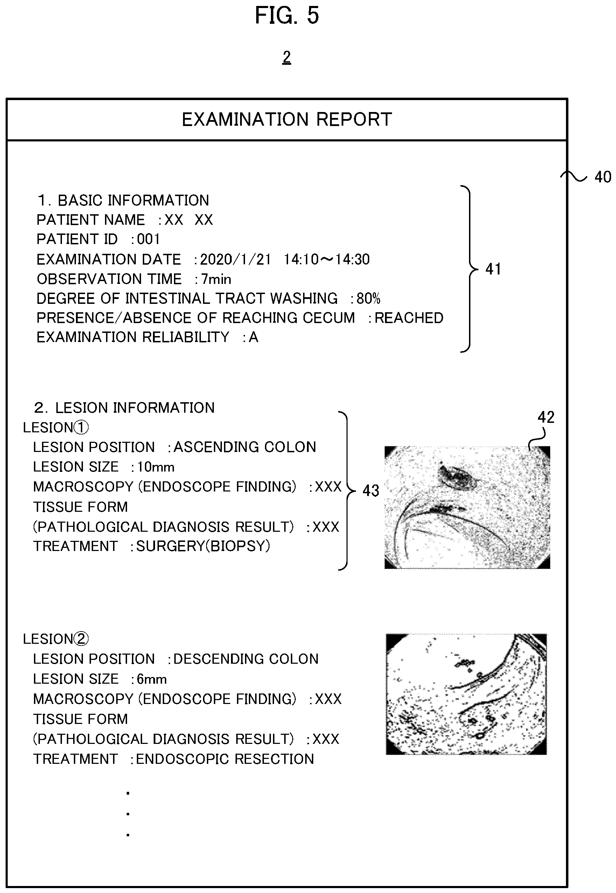

FIG. 5 shows a display example of the examination report. In this example, the information related to the examination quality and the lesion information for a certain patient's endoscopic examination are displayed.

Specifically, in the display example of FIG. 5, in addition to the basic information 41 representing the patient information and the examination quality, the lesion image 42 and the lesion information 43 are displayed in the display area 40.

The basic information 41 includes the patient information such as the patient name, the patient ID, and the examination date and time, and the information representing examination quality such as the observation time of the endoscopic examination, the degree of intestinal tract washing, the presence or absence of reaching the cecum, and the examination reliability.

Here, the observation time is the time required for the observation in endoscopic examination. Specifically, the time obtained by subtracting the treatment time from the elapsed time from the start time of the examination to the end time of the examination is displayed in the observation time.

The degree of intestinal tract washing represents a ratio of the range in the large intestine where there was no residue and observation of the large intestine was possible. In this case, though the degree of intestinal tract washing in the whole large intestine is displayed, the degree of intestinal tract washing for each region of the large intestine may be displayed. In addition, instead of the degree of intestinal tract washing, a ratio of the range in which it was not possible to observe the large intestine due to adhesion of stools or the like (also referred to as the "residue detection result") may be displayed.

The presence or absence of reaching the cecum represents whether the endoscope reached the caecum or not in endoscopy. Here, it is displayed "reached" if the endoscopic examination system 100 detects the ileocecal valve or the appendiceal orifice, and it is displayed "not reached" if the endoscopic examination system 100 does not detect the ileocecal valve or the appendiceal orifice.

The examination reliability is the comprehensive determination of the examination quality in the endoscopic examination based on the observation time, the degree of intestinal tract washing, and the presence or absence of reaching the cecum. Here, as an example, there are three conditions such as the observation time is 6 minutes or more, the degree of intestinal tract washing is equal to or more than a predetermined threshold value, and the presence or absence of reaching the cecum is "reached". "A" is displayed when all the three conditions are satisfied. "B" is displayed when two of those three conditions are satisfied. "C" is displayed when one of those three conditions is satisfied or none of those conditions is satisfied. Incidentally, the examination reliability may be determined using another standard or method.

The lesion image 42 shows the lesion image captured automatically by the AI determination unit 23 or the lesion image captured by the examiner in the endoscopic examination. Further, the lesion information 43 shows the analysis result of the lesion image 42 by the AI determination unit 23. Incidentally, the lesion information 43 may show the finding of the examiner, in addition to the analysis result of the lesion image 42. Here, the lesion position, the lesion size, the macroscopy (endoscope finding), the tissue form (pathological diagnosis result), the treatment, etc., are displayed. The lesion image 42 and the lesion information 43 are displayed for each lesion captured by the AI determination unit 23 or the examiner.

### [Image display processing]

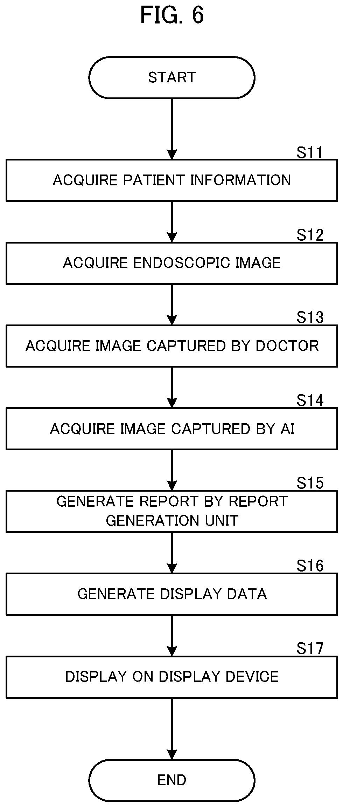

Next, display processing for performing the above-mentioned display will be described. FIG. 6 is a flowchart of display processing by the image processing device 1. This processing is realized by the processor 11 shown in FIG. 2, which executes a pre-prepared program and operates as each element shown in FIG. 3.

First, the report generation unit 24 acquires the patient information through the input unit 14 (step S11). The examination data generation unit 22 and the AI determination unit 23 acquire the endoscopic image Ic (step S12). Next, the examination data generation unit 22 acquires the lesion image captured by the doctor based on the endoscopic image Ic, and outputs the acquired image to the AI determination unit 23 (step S13). The AI determination unit 23 captures the lesion image independently on the basis of the endoscopic image Ic (step S14).

Next, the AI determination unit 23 determines the lesion size and the grade of malignancy of the lesion or the like based on the lesion image captured by the doctor and the lesion image captured independently by the AI determination unit 23, and outputs the determined the lesion information to the report generation unit 24. Also, based on the endoscopic image Ic, the AI determination unit 23 determines the presence or absence of reaching the cecum, calculates the observation time, and determines the degree of intestinal tract washing, and outputs the result to the report generation unit 24. The report generation unit 24 generates the report data using the information generated by the AI determination unit 23, and outputs the report data to the display data generation unit 25 (step S15).

The display data generation unit 25 generates the display data items as illustrated in FIG. 5 using the report data generated by the report generation unit 24 (step S16), and outputs the display data items to the display device 2 (step S17). Thus, the display like the display example of FIG. 5 is performed. Incidentally, step S14 may be performed prior to step S13, or may be performed simultaneously with step S13.

### (Modification)

In the above example embodiment, the AI determination unit 23 calculates the degree of washing of the entire large intestine, and displays it on the examination report. Instead, the AI determination unit may calculate the degree of washing for each region of the large intestine and display the degree of washing on the examination report. In addition, the name of the region whose degree of washing is lower than the standard value or the like may be displayed in the examination report.

### <Second example embodiment>

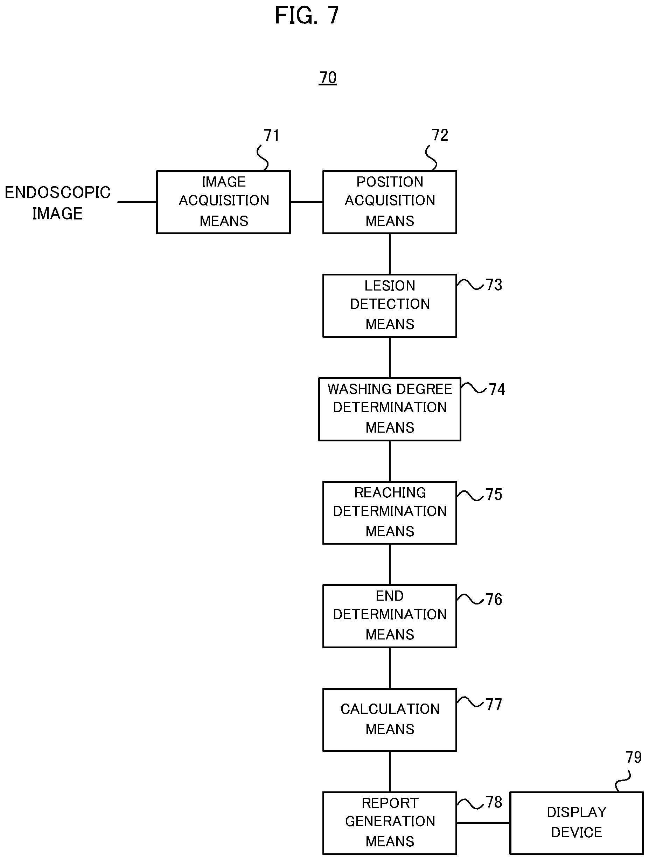

FIG. 7 is a block diagram showing a functional configuration of an information display device of a second example embodiment. The information display device 70 includes an image acquisition means 71, a position acquisition means 72, a lesion detection means 73, a washing degree determination means 74, a reaching determination means 75, an end determination means 76, a calculation means 77, a report generation means 78, and a display device 79.

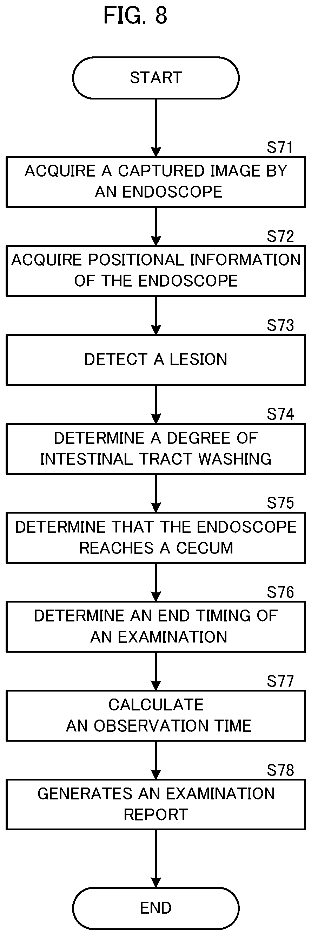

FIG. 8 is a flowchart of processing by the information display device of the second example embodiment. The image acquisition means 71 acquires a captured image by an endoscope (step S71). The position acquisition means 72 acquires positional information of the endoscope (step S72). The lesion detection means 73 detects a lesion from the captured image (step S73). The washing degree determination means 74 determines a degree of intestinal tract washing from the captured image (step S74). The reaching determination means 75 determines that the endoscope reaches a cecum from the captured image (step S75). The end determination means 76 determines an end timing of an examination by the endoscope (step S76). The calculation means 77 calculates an observation time based on a timing that the endoscope reaches the cecum and the end timing (step S77). The report generation means 78 generates an examination report including the observation time, the degree of intestinal tract washing, a presence or absence of reaching the cecum of the endoscope, and a detection result of the lesion including the positional information of the lesion (step S78). The generated examination report is displayed on the display device 79.

According to the information display device 70 of the second example embodiment, the examiner of the endoscopic examination can easily grasp information for the examination quality and the lesions analyzed by AI.

A part or all of the above example embodiments may also be described as in the following supplementary notes, but are not limited to:

### (Supplementary note 1)

An information processing device comprising:
an image acquisition means configured to acquire a captured image by an endoscope;
a position acquisition means configured to acquire positional information of the endoscope;
a lesion detection means configured to detect a lesion from the captured image;
a washing degree determination means configured to determine a degree of intestinal tract washing from the captured image;
a reaching determination means configured to determine that the endoscope reaches a cecum from the captured image;
an end determination means configured to determine an end timing of an examination by the endoscope;
a calculation means configured to calculate an observation time based on a timing that the endoscope reaches the cecum and the end timing; and
a report generation means configured to generate an examination report including the observation time, the degree of intestinal tract washing, a presence or absence of reaching the cecum of the endoscope, and a detection result of the lesion including the positional information of the lesion.

### (Supplementary note 2)

The information processing device according to Supplementary note 1, further comprising a reliability determination means configured to determine an examination reliability based on the observation time, the degree of intestinal tract washing, and the presence or absence of reaching the cecum of the endoscope,
wherein the examination report includes the examination reliability.

### (Supplementary note 3)

The information processing device according to Supplementary note 1 or 2, wherein the reaching determination means determines that the endoscope has reached the cecum when at least one of an ileocecal valve and an appendiceal orifice is detected in the captured image.

### (Supplementary note 4)

The information processing device according to any one of Supplementary note 1 to 3,
wherein the washing degree determination means determines the degree of intestinal tract washing for each region of a large intestine, and
wherein the examination report includes the degree of intestinal tract washing for each region of the large intestine.

### (Supplementary note 5)

The information processing device according to any one of Supplementary note 1 to 4, wherein the calculation means calculates the observation time by subtracting a treatment time in which a treatment for the lesion is performed, from the time from a timing that the endoscope reaches the cecum to the end timing.

### (Supplementary note 6)

The information processing device according to Supplementary note 5, wherein the calculation means calculates the treatment time based on the captured image.

### (Supplementary note 7)

The information processing device according to any one of Supplementary note 1 to 6, wherein the end determination means determines a timing at which the endoscope is pulled out of the large intestine based on the captured image as the end timing of the examination.

### (Supplementary note 8)

The information processing device according to any one of Supplementary note 1 to 6, wherein the end determination means determines a timing at which an instruction of ending the examination is inputted as the end timing of the examination.

### (Supplementary note 9)

An information processing method comprising:
acquiring a captured image by an endoscope;
acquiring positional information of the endoscope;
detecting a lesion from the captured image;
determining a degree of intestinal tract washing from the captured image;
determining that the endoscope reaches a cecum from the captured image;
determining an end timing of an examination by the endoscope;
calculating an observation time based on a timing that the endoscope reaches the cecum and the end timing; and
generating an examination report including the observation time, the degree of intestinal tract washing, a presence or absence of reaching the cecum of the endoscope, and a detection result of the lesion including the positional information of the lesion.

### (Supplementary note 10)

A recording medium storing a program, the program causing a computer to execute processing of:
acquiring a captured image by an endoscope;
acquiring positional information of the endoscope;
detecting a lesion from the captured image;
determining a degree of intestinal tract washing from the captured image;
determining that the endoscope reaches a cecum from the captured image;
determining an end timing of an examination by the endoscope;
calculating an observation time based on a timing that the endoscope reaches the cecum and the end timing; and
generating an examination report including the observation time, the degree of intestinal tract washing, a presence or absence of reaching the cecum of the endoscope, and a detection result of the lesion including the positional information of the lesion.

While the present disclosure has been described with reference to the example embodiments and examples, the present disclosure is not limited to the above example embodiments and examples. Various changes which can be understood by those skilled in the art within the scope of the present disclosure can be made in the configuration and details of the present disclosure.

### DESCRIPTION OF SYMBOLS

1 Image processing device
2 Display device
3 Endoscope
11 Processor
12 Memory
17 Database (DB)
21 Position detection unit
22 Examination data generation unit
23 AI determination unit
24 Report generation unit
100 Endoscopic examination system

## Claims

1. An information processing device comprising:
an image acquisition means configured to acquire a captured image by an endoscope;
a position acquisition means configured to acquire positional information of the endoscope;
a lesion detection means configured to detect a lesion from the captured image;
a washing degree determination means configured to determine a degree of intestinal tract washing from the captured image;
a reaching determination means configured to determine that the endoscope reaches a cecum from the captured image;
an end determination means configured to determine an end timing of an examination by the endoscope;
a calculation means configured to calculate an observation time based on a timing that the endoscope reaches the cecum and the end timing; and
a report generation means configured to generate an examination report including the observation time, the degree of intestinal tract washing, a presence or absence of reaching the cecum of the endoscope, and a detection result of the lesion including the positional information of the lesion.

2. The information processing device according to claim 1, further comprising a reliability determination means configured to determine an examination reliability based on the observation time, the degree of intestinal tract washing, and the presence or absence of reaching the cecum of the endoscope,
wherein the examination report includes the examination reliability.

3. The information processing device according to claim 1 or 2, wherein the reaching determination means determines that the endoscope has reached the cecum when at least one of an ileocecal valve and an appendiceal orifice is detected in the captured image.

4. The information processing device according to any one of claim 1 to 3,
wherein the washing degree determination means determines the degree of intestinal tract washing for each region of a large intestine, and
wherein the examination report includes the degree of intestinal tract washing for each region of the large intestine.

5. The information processing device according to any one of claim 1 to 4, wherein the calculation means calculates the observation time by subtracting a treatment time in which a treatment for the lesion is performed, from the time from a timing that the endoscope reaches the cecum to the end timing.

6. The information processing device according to claim 5, wherein the calculation means calculates the treatment time based on the captured image.

7. The information processing device according to any one of claim 1 to 6, wherein the end determination means determines a timing at which the endoscope is pulled out of the large intestine based on the captured image as the end timing of the examination.

8. The information processing device according to any one of claim 1 to 6, wherein the end determination means determines a timing at which an instruction of ending the examination is inputted as the end timing of the examination.

9. An information processing method comprising:
acquiring a captured image by an endoscope;
acquiring positional information of the endoscope;
detecting a lesion from the captured image;
determining a degree of intestinal tract washing from the captured image;
determining that the endoscope reaches a cecum from the captured image;
determining an end timing of an examination by the endoscope;
calculating an observation time based on a timing that the endoscope reaches the cecum and the end timing; and
generating an examination report including the observation time, the degree of intestinal tract washing, a presence or absence of reaching the cecum of the endoscope, and a detection result of the lesion including the positional information of the lesion.

10. A recording medium storing a program, the program causing a computer to execute processing of:
acquiring a captured image by an endoscope;
acquiring positional information of the endoscope;
detecting a lesion from the captured image;
determining a degree of intestinal tract washing from the captured image;
determining that the endoscope reaches a cecum from the captured image;
determining an end timing of an examination by the endoscope;
calculating an observation time based on a timing that the endoscope reaches the cecum and the end timing; and
generating an examination report including the observation time, the degree of intestinal tract washing, a presence or absence of reaching the cecum of the endoscope, and a detection result of the lesion including the positional information of the lesion.
